# EUROPEAN PATENT SPECIFICATION

(11) **EP 3 954 996 B1**
(45) Date of publication and mention of the grant of the patent: **18.12.2024**
(21) Application number: 21185369.2
(22) Date of filing: 13.07.2021
(51) Int. Cl.: G01N 33/532, G01N 33/569, G01N 33/58

(54) **BIOLOGICAL NANOPARTICLE DETECTING METHOD WITH HIGH SENSITIVITY**
VERFAHREN MIT HOHER EMPFINDLICHKEIT ZUR DETEKTION BIOLOGISCHER NANOPARTIKEL
PROCÉDÉ DE DÉTECTION DE NANOPARTICULES BIOLOGIQUES À HAUTE SENSIBILITÉ

(30) Priority: 10.08.2020 CN 202010797874
(43) Date of publication of application: 16.02.2022
(73) Proprietor: Nanjing Medical University, Nanjing, Jiangsu (CN)
(72) Inventor: FANG, Yimin, Nanjing (CN); CHEN, Shan, Nanjing (CN); JIANG, Tao, Nanjing (CN); HUANG, Zongxiong, Nanjing (CN); YIN, Congcong, Nanjing (CN)
(74) Representative: Zenz Patentanwälte Partnerschaft mbB

(56) References cited:
- HE FANG ET AL: "Quantification of Exosome Based on a Copper-Mediated Signal Amplification Strategy", ANALYTICAL CHEMISTRY, vol. 90, no. 13, 12 June 2018 (2018-06-12), US, pages 8072 - 8079, XP055864059, ISSN: 0003-2700, DOI: 10.1021/acs.analchem.8b01187
- CHENG NAN ET AL: "Recent Advances in Biosensors for Detecting Cancer-Derived Exosomes", TRENDS IN BIOTECHNOLOGY, ELSEVIER PUBLICATIONS, CAMBRIDGE, GB, vol. 37, no. 11, 26 October 2019 (2019-10-26), pages 1236 - 1254, XP085889870, ISSN: 0167-7799, [retrieved on 20191026], DOI: 10.1016/J.TIBTECH.2019.04.008
- XU HUIYING ET AL: "Advances in biosensing technologies for analysis of cancer-derived exosomes", TRAC TRENDS IN ANALYTICAL CHEMISTRY, ELSEVIER, AMSTERDAM, NL, vol. 123, 13 December 2019 (2019-12-13), XP086022419, ISSN: 0165-9936, [retrieved on 20191213], DOI: 10.1016/J.TRAC.2019.115773
- CHEN SHAN ET AL: "Triethylamine as a complexing reagent for highly efficient naked-eyes copper ions sensing - A new catalytic pathway for ultrasensitive detection", SENSORS AND ACTUATORS B: CHEMICAL, ELSEVIER BV, NL, vol. 305, 11 November 2019 (2019-11-11), XP085968832, ISSN: 0925-4005, [retrieved on 20191111], DOI: 10.1016/J.SNB.2019.127373
- CHEN SHAN ET AL: "Fast and Ultrasensitive Visual Detection of Exosomes in Body Fluids for Point-of-Care Disease Diagnosis", ANALYTICAL CHEMISTRY, vol. 93, no. 29, 13 July 2021 (2021-07-13), US, pages 10372 - 10377, XP055863665, ISSN: 0003-2700, DOI: 10.1021/acs.analchem.1c02136

## Description

### TECHNICAL FIELD

The present disclosure relates to the technical field of analytical chemistry and disease diagnosis, and in particular to a biological nanoparticle detection method with high-sensitivity.

### BACKGROUND

Exosomes are nano-sized cystic vesicles secreted by cells, which play an important role in cell-cell communication. In normal physiological and pathological conditions, almost all cells can secrete exosomes, but the number of exosomes secreted by cancerous cells is usually several orders of magnitude higher than that secreted by normal cells.

The size of exosomes ranges from about 30 nm to 150 nm. They comprise a variety of membrane proteins on the surface and nucleic acids, active enzymes and cytoplasmic substrates therein. Exosomes also comprise many proteins, and these proteins reflect the phenotype and physiological state of the cells, and are highly heterogeneous. The above-mentioned characteristics of exosomes can inform the relevant physiological states and pathological processes of many diseases, especially cancers. Therefore, the sensitive recognition of exosomes secreted by cells is of great significance for the biological research and clinical disease diagnosis.

Viruses are tiny life forms that can utilize nutrients in host cells and replicate their own life components such as nucleic acids and proteins. Viruses cause damage to human cells and tissues. For example, influenza viruses, HIV, and hepatitis viruses are common viruses. Most viruses are highly infectious. Effective and timely detection of the viruses and isolation of the source of infection to cut off the routes of infection are of great practical significance for arresting the spread of viruses and for the diagnosis and treatment of diseases.

Due to the small size of biological nanoparticles, for example, the size of viruses ranges from 60 nm to 140 nm, and the size of exosomes ranges from about 30 nm to 150 nm, they cannot be detected under ordinary optical microscopes. Fluorescence staining or flow cytometry is usually used to analyze and detect biological nanoparticles. However, the complicated fluorescence staining process and the weak light scattering of biological nanoparticles such as exosomes and viruses limit the use of these two methods in the detection of biological nanoparticles such as exosomes and viruses. Accordingly, in view of the above-mentioned technical problems, for nano-sized exosomes, viruses and other biological nanoparticles, visual analysis under a transmission electron microscope and nanoparticle tracking analysis are employed in the prior art. However, the transmission electron microscope and nanoparticle tracking analysis instrument are expensive, and the cost is about 500 RMB for each test of a biological sample such as exosomes and viruses. Moreover, before the biological nanoparticles such as exosomes and viruses are detected under a transmission electron microscope, they need to be stained; and the nanoparticle tracking analysis method requires a complicated separation and purification process. CHENG NAN ET AL ("Recent Advances in Biosensors for Detecting Cancer-Derived Exosomes", TRENDS IN BIOTECHNOLOGY, ELSEVIER PUBLICATIONS, CAMBRIDGE, GB, vol. 37, no. 11, 26 October 2019, pages 1236-1254) discloses a method for detecting exosomes comprising reacting copper nanoparticles with an aptamer, and after binding of said aptamer to the exosome, fluorescence was achieved through the acidolysis of transforming CuO NP into copper (II) ions (Cu2+) and the reduction of Cu2+ into fluorescent CuNPs by sodium ascorbate in the presence of poly(thymine). He Fang ET AL ("Quantification of Exosome Based on a Copper-Mediated Signal Amplification Strategy", Analytical Chemistry, vol. 90, no. 13, 12 June 2018, pages 8072-8079) makes a similar disclosure for detecting exosomes. XU HUIYING ET AL ("Advances in biosensing technologies for analysis of cancer-derived exosomes", TRAC TRENDS IN ANALYTICAL CHEMISTRY, ELSEVIER, AMSTERDAM, NL, vol. 123, 13 December 2019) similarly discloses a method for detecting exosomes wherein exosomes are first captured by cholesterol-modified magnetic beads, then the bead-binding exosomes and CD63 aptamer-modified copper oxide nanoparticles (CuO NPs) can form sandwich complexes through the special recognition of CD63 aptamer, and after magnetic separation, the acidolysis transforms CuO NP into Cu2+, which can be further reduced to fluorescent copper nanoparticles (CuNPs).

### SUMMARY

To overcome the technical defects in the prior art of complicated pretreatment process before the detection of biological nanoparticles such as exosomes and viruses, expensive detection equipment and detection cost, as well as inability to distinguish interfering particles, an object of the present disclosure provides a simple, convenient and rapid biological nanoparticle detection method with high-sensitivity such as an exosome and a virus by specifically binding a labeling protein to the biological nanoparticle, to achieve the rapid and high-sensitivity detection of the biological nanoparticle such as an exosome and a virus.

To achieve the above objective, a biological nanoparticle detection method with high-sensitivity is provided in the present disclosure, which includes the following steps:

Step S1: reacting a copper compound nanoparticle with a surface membrane protein aptamer having a sulfhydryl group of the biological nanoparticle to obtain a copper compound-membrane protein aptamer conjugate, wherein the copper compound nanoparticle is one or more selected from a group consisting of: cupric sulfide, cuprous oxide, and cuprous sulfide;

Step S2: filtering a biological nanoparticle solution containing the biological nanoparticle through a first filter membrane, and adding the copper compound-membrane protein aptamer conjugate to the filtered solution, to obtain a biological nanoparticle-copper compound conjugate after reaction;

Step S3: adding a surfactant to the reaction solution obtained in Step S2, filtering through a second filter membrane, and washing the second filter membrane with PBS to obtain a third filter membrane containing the biological nanoparticle-copper compound conjugate, wherein a pore size of the second filter membrane is 50 nm; and

Step S4: adding a AgNO₃ solution to the third filter membrane obtained in Step S3 and reacting; and then adding a mixed solution of triethylamine hydrochloride, hydrogen peroxide and 3,3',5,5'-tetramethylbenzidine, reacting for development, and observing the color change of the filter membrane visually by naked eyes or by means of a camera.

Preferably, the biological nanoparticle is an exosome or a virus.

Preferably, in Step S1, a size of the copper compound nanoparticle is 5 to 50 nm, and the surface membrane protein aptamer having a sulfhydryl group is one or more selected from a groups consisting of: CD63 aptamer, CD81 aptamer, CD9 aptamer, EpCAM aptamer, HER2 aptamer, MUC1 aptamer, and PSMA aptamer; the reaction time of the copper compound nanoparticle with the surface membrane protein aptamer is 8 to 24 hrs; and a pore size of the first filter membrane is 200 nm.

Preferably, the reaction time of the biological nanoparticle solution with the copper compound-membrane protein aptamer conjugate in Step S2 is 0.5 to 10 hrs.

Preferably, a volume of the biological nanoparticle solution in Step S2 is adjustable, when the concentration of the biological nanoparticle solution is low, the volume of the biological nanoparticle solution is increased to improve the sensitivity.

Preferably, the surfactant in Step S3 is one or more selected from a group consisting of: sodium dodecyl sulfate, cetyltrimethylammonium bromide, and polyvinylpyrrolidone, and the concentration of the surfactant is in the range of 0.1 to 2.0%.

Preferably, the AgNO₃ solution in Step S4 has a concentration of 10⁻⁵-10⁻³ M and a volume of 10 to 50 uL, and the reaction time of substance on a surface of the third filter membrane with the AgNO₃ solution is in the range of 5 to 10 min, the concentration of triethylamine hydrochloride is 0.05 to 0.2 M, the concentration of hydrogen peroxide is 0.1 to 0.5 M, the concentration of 3,3',5,5'-tetramethylbenzidine is 0.1 to 1.0 mM, and the reaction time is 5 to 30 min.

Compared with the prior art, the technical solution of the present disclosure has the following advantages:

The technical solution of the present disclosure is based on the highly specific antigen-antibody reaction and the high-efficiency catalytic reaction of copper-amine complexation, and can be used in the high-sensitivity detection of biological nanoparticles such as exosomes and viruses, by visual colorimetric method or by comparison with photos taken by a camera. In this way, high-sensitivity detection of an exosome solution having a concentration as low as 2.5 × 10⁵ counts/mL is easily achieved without the aid of any instruments.

Compared with the prior art, in the technical solution of the present disclosure, a labeling protein is specifically bound to a biological nanoparticle such as an exosome and a virus, and a copper compound-biological nanoparticle is enriched by filtering through a filter membrane, which can not only eliminate the interference from proteins and small molecules in an actual sample, but also achieve the rapid and high-sensitivity detection of the biological nanoparticle such as an exosome and s virus.

Moreover, the detection method according to the technical solution of the present disclosure has the advantages of low cost, high reaction efficiency, and excellent stability, and can be widely used in the detection of biological nanoparticles such as exosomes and viruses, and in the diagnosis of disease, especially cancer detection.

### BRIEF DESCRIPTION OF THE DRAWINGS

In order to more clearly explain the technical solutions in the embodiments of the present disclosure or in the prior art, the drawings used in the description of the embodiments or the prior art will be briefly described below. Evidently, the drawings depicted below are merely some embodiments of the present disclosure, and those skilled in the art can obtain other drawings based on the structures shown in these drawings without any creative efforts.
FIG. 1 is a diagram showing a comparison of the changes in color between the filter membrane in the control group and the filter membrane obtained after the reaction with a solution of an exosome of 10⁹ counts/mL in a first Example of the present disclosure;
FIG. 2 is a diagram showing a comparison of the changes in color between the filter membrane in the control group and the filter membranes obtained after the reaction with various concentrations of exosome solutions in a second Example of the present disclosure; and
FIG. 3 is a diagram showing a comparison of the changes in color between the filter membrane in the control group and the filter membrane obtained after the reaction with a solution of an exosome of 1 × 10⁷ counts/mL in a third Example of the present disclosure.

The objects, functional characteristics and advantages of the present disclosure will be further described in combination with the embodiments and with reference to the accompanying drawings.

### DESCRIPTION OF THE EMBODIMENTS

The technical solutions in the embodiments of the present disclosure will be described clearly and fully with reference to the accompanying drawings in the embodiments of the present disclosure.

It should be noted that if there are directional indications (such as on, below, left, right, front, back...) involved in the embodiments of the present disclosure, these directional indications are only used to explain the relative positional relationship and movement of various components in a specific posture (as shown in the figures). If the specific posture changes, the directional indications will change accordingly.

In addition, if there are descriptions "first", and "second", etc. in the embodiments of the present disclosure, the descriptions "first" and "second" are used herein merely for the purposes of description, and are not intended to indicate or imply the relative importance or implicitly point out the number of the indicated technical feature. Therefore, the features defined by "first", and "second" may explicitly or implicitly include at least one of the features. In addition, the technical solutions in various embodiments can be combined with each other, on the condition that the combinations can be accomplished by those of ordinary skill in the art. When a combination of technical solutions is contradictory or cannot be achieved, it is considered that such a combination of technical solutions does not exist, and does not fall within the protection scope of the present disclosure.

The present disclosure provides a method for high-sensitivity detection of an exosome.

### First Example

In this example of the present disclosure, 1 mL of a CuS nanoparticle solution with a concentration of 10¹³ counts/mL was added to 20 uL of a solution of CD63 aptamer having a sulfhydryl group. Then, a 2 M sodium chloride solution was gradually added to give a final sodium chloride concentration of 0.1 M in the solution system. After 8 hrs of reaction, the reaction solution was centrifuged and washed to obtain CuS nanoparticles bearing CD63 aptamer.

20 uL of CuS nanoparticles bearing CD63 aptamer was added to 1 mL of an exosome solution with a concentration of 10⁹ counts/mL and reacted for half an hour. Then 0.1% sodium dodecyl sulfate was added, and the solution was filtered through a filter membrane with a pore size of 50 nm and washed three times with PBS, to obtain a filter membrane containing exosomes-CuS. 10 uL of a AgNO₃ solution having a concentration of 1.0 x 10⁻³ M was added to the filter membrane and reacted for 5 min.

A newly prepared 3,3',5,5'-tetramethylbenzidine (TMB) solution and 5 uL of a newly prepared 10 mol/L hydrogen peroxide solution were added to 500 uL of a triethylamine hydrochloride solution, and mixed uniformly to prepare a detection solution.

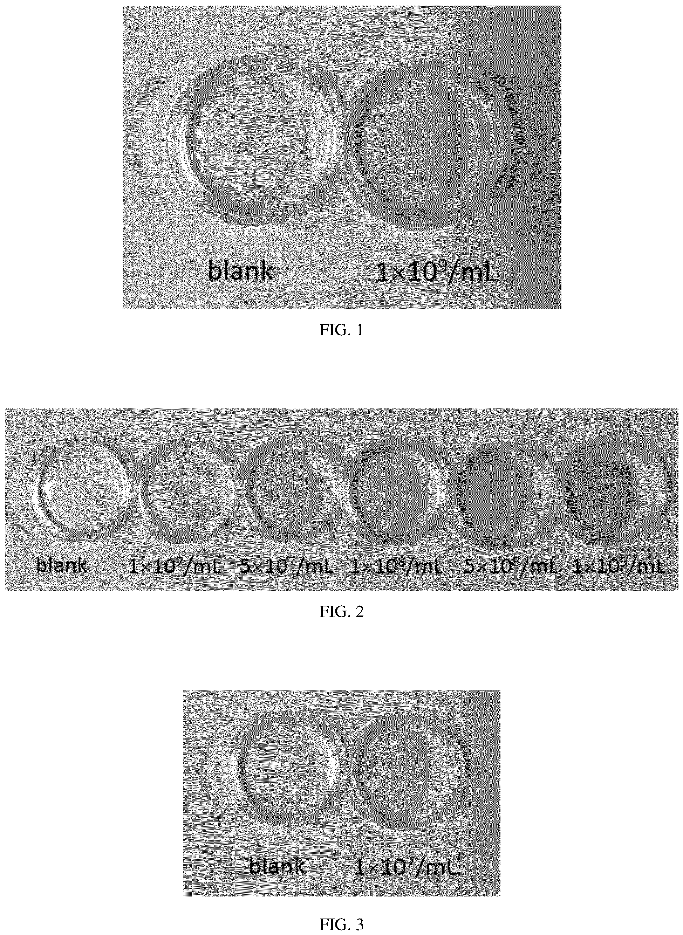

The detection solution was added to the control group (a filter membrane obtained by performing the above experiment with a control solution without exosomes) and the filter membrane containing exosomes of 10⁹ counts/mL. After standing for 5 min, the change in color between the filter membranes was observed and detected visually by naked eyes or by taking photos with a camera. The changes in color between the control group (the filter membrane obtained by performing the above experiment with the control solution without exosomes) and the filter membrane containing exosomes of 10⁹counts/mL is shown in FIG. 1. As shown in FIG. 1, the filter membrane with exosomes has a very significant difference in color compared to the filter membrane without exosomes.

### Second Example

In this example of the present disclosure, 1 mL of a CuS nanoparticle solution with a concentration of 10¹³ counts/mL was added to 20 uL of a solution of CD63 aptamer having a sulfhydryl group. After 8 hrs of reaction, the reaction solution was centrifuged and washed to obtain CuS nanoparticles bearing CD63 aptamer.

20 uL of CuS nanoparticles bearing CD63 aptamer was added respectively to 1 mL of an exosome solution with a concentration of 1 × 10⁷ counts/mL, 5 × 10⁷ counts/mL, 1 × 10⁸ counts/mL, 5 x 10⁸ counts/mL, and 1.0 x 10⁹ counts/mL and reacted for half an hour. Then 0.1% cetyltrimethyl ammonium bromide was added, and the solution was filtered through a filter membrane with a pore size of 50 nm and washed three times with PBS, to obtain a filter membrane containing exosomes-CuS. 10 uL of a AgNO₃ solution having a concentration of 5.0 x 10⁻⁴ M was added to the filter membrane and reacted for 10 min.

A newly prepared 3,3',5,5'-tetramethylbenzidine (TMB) solution and 5 uL of a newly prepared 10 mol/L hydrogen peroxide solution were added to 500 uL of a triethylamine hydrochloride solution, and mixed uniformly to prepare a detection solution.

The detection solution was added to the control group (a filter membrane obtained by performing the above experiment with a control solution without exosomes) and the filter membranes containing different concentrations of exosome (1 × 10⁷ counts/mL, 5 x 10⁷ counts/mL, 1 × 10⁸ counts/mL, 5 × 10⁸ counts/mL, and 1.0 x 10⁹ counts/mL). After 5 min of reaction, the changes in color between the filter membranes was detected by visual colorimetric method or by taking photos with a camera. The change in color is shown in FIG. 2. As shown in FIG. 2, as the exosome concentration in the sample solution continues to increase, the color of the filter membrane continues to deepen, such that direct observation with the naked eyes is achieved.

### Third Example

In this example of the present disclosure, 50 mL of a CuS nanoparticle solution with a concentration of 10¹³ counts/mL was added to 1 mL of a solution of CD63 aptamer having a sulfhydryl group. After 8 hrs of reaction, the reaction solution was centrifuged and washed to obtain CuS nanoparticles bearing CD63 aptamer.

40 mL of CuS nanoparticles bearing CD63 aptamer was added to 200 mL of an exosome solution with a concentration of 1.0 x 10⁷ counts/mL and reacted for half an hour. Then 0.3% sodium dodecyl sulfate was added, and the solution was filtered through a filter membrane with a pore size of 50 nm and washed three times with PBS, to obtain a filter membrane containing exosome-CuS. 10 uL of a AgNO₃ solution having a concentration of 5.0 x 10⁻⁴ M was added to the filter membrane and reacted for 5 min.

A newly prepared 3,3',5,5'-tetramethylbenzidine (TMB) solution and 5 uL of a newly prepared 10 mol/L hydrogen peroxide solution were added to 250 uL of a triethylamine hydrochloride solution, and mixed uniformly to prepare a detection solution.

The detection solution was added to the control group (a filter membrane obtained by performing the above experiment with a control solution without exosomes) and the filter membrane containing exosomes of 1.0 x 10⁷ counts/mL. After 5 min of reaction, the change in color between the filter membranes was detected by visual colorimetric method or by taking photos with a camera. The changes in color is shown in FIG. 3.

### Example 4

In this example of the present disclosure, 1 mL of a CuS nanoparticle solution with a concentration of 10¹³ counts/mL was added to 20 uL of a solution of CD63 aptamer having a sulfhydryl group. Then, a 2 M sodium chloride solution was gradually added to give a final sodium chloride concentration of 0.1 M in the solution system. After 8 hrs of reaction, the reaction solution was centrifuged and washed to obtain CuS nanoparticles bearing CD63 aptamer.

100 uL of CuS nanoparticles bearing CD63 aptamer was added to 5 mL of an exosome solution with a concentration of 10⁷ counts/mL and reacted for half an hour. Then 0.5% sodium dodecyl sulfate was added, and the solution was filtered through a filter membrane with a pore size of 50 nm and washed three times with PBS, to obtain a filter membrane containing exosome-CuS. 10 uL of a AgNO₃ solution having a concentration of 1.0 x 10⁻³ M was added to the filter membrane and reacted for 5 min.

A newly prepared 3,3',5,5'-tetramethylbenzidine (TMB) solution and 10 uL of a newly prepared 10 mol/L hydrogen peroxide solution were added to 400 uL of a triethylamine hydrochloride solution, and mixed uniformly to prepare a detection solution.

The detection solution was added to the control group (a filter membrane obtained by performing the above experiment with a control solution without exosomes) and the filter membrane containing exosomes of 10⁷ counts/mL. After standing for 5 min, the changes in color between the filter membranes in the control group and the experiment group was detected by visual colorimetric method or by taking photos with a camera.

### Example 5

In this example of the present disclosure, 0.01 g of sodium dodecyl sulfate (SDS) was added to 1.0 mL of a CuS solution, and then 30 uL of 100 uM Thiol-Virus Aptamer and 10 uL of 2.5 mM tris(2-carboxyethyl)phosphine (TCEP) were added and reacted for 30 min to obtain a mixed solution. A 2 M NaCl solution was gradually added to give a final NaCl concentration of 0.1 M in the mixed solution. After 12 hrs of reaction, excess Thiol-Virus Aptamer was removed by centrifugation and washing three times with PBS, to obtain CuS-DNA complex particles, which was made up to 1.0 mL with PBS and stored in a freezer at 4°C.

1.0 mL of a solution containing a certain concentration of highly pathogenic H5N1 avian influenza virus was added to 20 uL of the above-mentioned CuS-DNA solution. After mixing and reacting for 1 hr, 0.5% SDS was added, and the solution was passed through a filter membrane having a pore size of 60 nm to obtain a filter membrane containing virus-CuS complex particles. Then the filter membrane was taken out and 20 uL of 10⁻⁴ M AgNO₃ was added and reacted for 5 min.

A newly prepared 3,3',5,5'-tetramethylbenzidine (TMB) solution and 10 uL of a newly prepared 10 mol/L hydrogen peroxide solution were added to 400 uL of a triethylamine hydrochloride solution, and mixed uniformly to prepare a detection solution.

The detection solution was added to the control group (a filter membrane obtained by performing the above experiment with a control solution without viruses) and the filter membrane containing viruses of 10⁷ counts/mL. After standing for 5 min, the changes in color between the filter membranes in the control group and the experiment group was detected by visual colorimetric method or by taking photos with a camera.

### Example 6

In this example of the present disclosure, 0.01 g of sodium dodecyl sulfate (SDS) was added to 1.0 mL of a CuS solution, and then 30 uL of 100 uM Thiol-Virus Aptamer and 10 uL of 2.5 mM tris(2-carboxyethyl)phosphine (TCEP) were added and reacted for 30 min to obtain a mixed solution. A 2 M NaCl solution was gradually added to give a final NaCl concentration of 0.1 M in the mixed solution. After 12 hrs of reaction, excess Thiol-Virus Aptamer was removed by centrifugation and washing three times with PBS, to obtain CuS-DNA complex particles, which was made up to 1.0 mL with PBS and stored in a freezer at 4°C.

1.0 mL of a solution containing a certain concentration of highly pathogenic H5N1 avian influenza virus was added to 20 uL of the above-mentioned CuS-DNA solution. After mixing and reacting for 1 hr, 0.5% SDS was added, and the solution was passed through a filter membrane having a pore size of 70 nm to obtain a filter membrane containing virus-CuS complex particles. Then the filter membrane was taken out and 20 uL of 10⁻⁴ M AgNO₃ was added and reacted for 5 min.

A newly prepared 3,3',5,5'-tetramethylbenzidine (TMB) solution and 10 uL of a newly prepared 10 mol/L hydrogen peroxide solution were added to 400 uL of a triethylamine hydrochloride solution, and mixed uniformly to prepare a detection solution.

The detection solution was added to the control group (a filter membrane obtained by performing the above experiment with a control solution without viruses) and the filter membrane containing exosomes of 10⁸ counts/mL. After standing for 5 min, the changes in color between the filter membranes in the control group and the experiment group was detected by visual colorimetric method or by taking photos with a camera.

### Example 7

In this example of the present disclosure, 0.01 g of sodium dodecyl sulfate (SDS) was added to 1.0 mL of a CuS solution, and then 30 uL of 100 uM Thiol-Virus Aptamer and 10 uL of 2.5 mM tris(2-carboxyethyl)phosphine (TCEP) were added and reacted for 30 min to obtain a mixed solution. A 2 M NaCl solution was gradually added to give a final NaCl concentration of 0.1 M in the mixed solution. After 12 hrs of reaction, excess Thiol-Virus Aptamer was removed by centrifugation and washing three times with PBS, to obtain CuS-DNA complex particles, which was made up to 1.0 mL with PBS and stored in a freezer at 4°C.

1.0 mL of a solution containing a certain concentration of highly pathogenic H5N1 avian influenza virus was added to 20 uL of the above-mentioned CuS-DNA solution. After mixing and reacting for 1 hr, 1.0% SDS was added, and the solution was passed through a filter membrane having a pore size of 70 nm to obtain a filter membrane containing virus-CuS complex particles. Then the filter membrane was taken out and 20 uL of 10⁻⁴ M AgNO₃ was added and reacted for 5 min.

A newly prepared 3,3',5,5'-tetramethylbenzidine (TMB) solution and 10 uL of a newly prepared 20 mol/L hydrogen peroxide solution were added to 400 uL of a triethylamine hydrochloride solution, and mixed uniformly to prepare a detection solution.

The detection solution was added to the control group (a filter membrane obtained by performing the above experiment with a control solution without viruses) and the filter membrane containing viruses of 5 * 10⁸ counts/mL. After standing for 5 min, the change in color between the filter membranes in the control group and the experiment group was detected by visual colorimetric method.

## Claims

1. A biological nanoparticle detection method with high-sensitivity, comprising the following steps:
Step S1: reacting a copper compound nanoparticle with a surface membrane protein aptamer having a sulfhydryl group to obtain a copper compound-membrane protein aptamer conjugate,
**characterized in that**:
wherein the copper compound nanoparticle is one or more selected from a group consisting of: cupric sulfide, cuprous oxide, and cuprous sulfide;
Step S2: filtering a biological nanoparticle solution containing the biological nanoparticle through a first filter membrane, and adding the copper compound-membrane protein aptamer conjugate to the filtered solution, to obtain a biological nanoparticle-copper compound conjugate after reaction;
Step S3: adding a surfactant to the reaction solution obtained in Step S2, filtering through a second filter membrane, and washing the second filter membrane with PBS to obtain a filter membrane containing the biological nanoparticle-copper compound conjugate, wherein a pore size of the second filter membrane is 50 nm; and
Step S4: adding a AgNO₃ solution to the filter membrane obtained in Step S3 and reacting: and then adding a mixed solution of triethylamine hydrochloride, hydrogen peroxide and 3,3',5,5'-tetramethylbenzidine, reacting for development, and observing the color change of the filter membrane visually by naked eyes or by means of a camera.

2. The method according to claim 1, wherein the biological nanoparticle is an exosome or a virus.

3. The method according to claim 1, wherein in Step S1, a size of the copper compound nanoparticle is 5 to 50 nm, and the surface membrane protein aptamer having a sulfhydryl group is one or more selected from a groups consisting of: CD63 aptamer, CD81 aptamer, CD9 aptamer, EpCAM aptamer, HER2 aptamer, MUC1 aptamer, and PSMA aptamer; the reaction time of the copper compound nanoparticle with the surface membrane protein aptamer is 8 to 24 hrs; and a pore size of the first filter membrane is 200 nm.

4. The method according to claim 1, wherein the reaction time of the biological nanoparticle solution with the copper compound-membrane protein aptamer conjugate in Step S2 is 0.5 to 10 hrs.

5. The method according to claim 1, wherein a volume of the biological nanoparticle solution in Step S2 is adjustable, when the concentration of the biological nanoparticle solution is low, the volume of the biological nanoparticle solution is increased to improve the sensitivity.

6. The method according to claim 1, wherein the surfactant in Step S3 is one or more selected from a group consisting of: sodium dodecyl sulfate, cetyltrimethylammonium bromide, and polyvinylpyrrolidone, and the concentration of the surfactant is in the range of 0.1 to 2.0%.

7. The method according to claim 1, wherein the AgNO₃ solution in Step S4 has a concentration of 10⁻⁵ to 10⁻³ M and a volume of 10 to 50 uL, and the reaction time of substance on a surface of the filter membrane with the AgNO₃ solution is in the range of 5 to 10 min, the concentration of triethylamine hydrochloride is 0.05 to 0.2 M, the concentration of hydrogen peroxide is 0.1 to 0.5 M, the concentration of 3,3',5,5'-tetramethylbenzidine is 0.1 to 1.0 mM, and the reaction time is 5 to 30 min.

## Patentansprüche

1. Ein Verfahren zum hochempfindlichen Nachweis biologischer Nanopartikel, das die folgenden Schritte umfasst:
Schritt S1: Umsetzen eines Nanopartikels einer Kupferverbindung mit einem Oberflächenmembranprotein-Aptamer mit einer Sulfhydrylgruppe, um ein Konjugat aus einer Kupferverbindung und einem Membranprotein-Aptamer zu erhalten, **dadurch gekennzeichnet, dass**:
das Kupferverbindungs-Nanopartikel eines oder mehrere ist, ausgewählt aus einer Gruppe, bestehend aus: Kupfersulfid, Kupfer(I)-oxid und Kupfer(I)-sulfid;
Schritt S2: Filtrieren einer biologischen Nanopartikel-Lösung, die das biologische Nanopartikel enthält, durch eine erste Filtermembran und Zugeben des Kupferverbindungs-Membranprotein-Aptamer-Konjugats zu der filtrierten Lösung, um nach der Reaktion ein biologisches Nanopartikel-Kupferverbindungs-Konjugat zu erhalten;
Schritt S3: Zugabe eines Tensids zu der in Schritt S2 erhaltenen Reaktionslösung, Filtern durch eine zweite Filtermembran und Waschen der zweiten Filtermembran mit PBS, um eine Filtermembran zu erhalten, die das biologische Nanopartikel-Kupferverbindungskonjugat enthält, wobei die Porengröße der zweiten Filtermembran 50 nm beträgt; und
Schritt S4: Zugabe einer AgNO₃-Lösung zu der in Schritt S3 erhaltenen Filtermembran und Reaktion; und dann Zugabe einer gemischten Lösung aus Triethylaminhydrochlorid, Wasserstoffperoxid und 3,3',5,5'-Tetramethylbenzidin, Reaktion zur Entwicklung und visuelle Beobachtung der Farbänderung der Filtermembran mit bloßem Auge oder mittels einer Kamera.

2. Das Verfahren nach Anspruch 1, wobei das biologische Nanopartikel ein Exosom oder ein Virus ist.

3. Das Verfahren nach Anspruch 1, wobei in Schritt S1 die Größe des Kupferverbindungs-Nanopartikels 5 bis 50 nm beträgt und das Oberflächenmembranprotein-Aptamer mit einer Sulfhydrylgruppe eines oder mehrere ist, ausgewählt aus einer Gruppe bestehend aus: CD63-Aptamer, CD81-Aptamer, CD9-Aptamer, EpCAM-Aptamer, HER2-Aptamer, MUC1-Aptamer und PSMA-Aptamer ausgewählt ist; die Reaktionszeit des Kupferverbindungs-Nanopartikels mit dem Oberflächenmembranprotein-Aptamer 8 bis 24 Stunden beträgt; und eine Porengröße der ersten Filtermembran 200 nm beträgt.

4. Das Verfahren nach Anspruch 1, wobei die Reaktionszeit der Lösung der biologischen Nanopartikel mit dem Kupferverbindungs-Membranprotein-Aptamer-Konjugat in Schritt S2 0,5 bis 10 Stunden beträgt.

5. Das Verfahren nach Anspruch 1, wobei ein Volumen der Lösung der biologischen Nanopartikel in Schritt S2 einstellbar ist, wobei das Volumen der Lösung der biologischen Nanopartikel erhöht wird, wenn die Konzentration der Lösung der biologischen Nanopartikel niedrig ist, um die Empfindlichkeit zu verbessern.

6. Das Verfahren nach Anspruch 1, wobei das Tensid in Schritt S3 eines oder mehrere ist, ausgewählt aus einer Gruppe, bestehend aus: Natriumdodecylsulfat, Cetyltrimethylammoniumbromid und Polyvinylpyrrolidon, und die Konzentration des Tensids im Bereich von 0,1 bis 2,0 % liegt.

7. Das Verfahren nach Anspruch 1, wobei die AgNO₃-Lösung in Schritt S4 eine Konzentration von 10⁻⁵ bis 10⁻³ M und ein Volumen von 10 bis 50 uL aufweist und die Reaktionszeit der Substanz auf einer Oberfläche der Filtermembran mit der AgNO₃-Lösung im Bereich von 5 bis 10 min liegt, die Konzentration von Triethylaminhydrochlorid 0,05 bis 0,2 M, die Konzentration von Wasserstoffperoxid 0,1 bis 0,5 M, die Konzentration von 3,3',5,5'-Tetramethylbenzidin 0,1 bis 1,0 mM und die Reaktionszeit 5 bis 30 min beträgt.

## Revendications

1. Méthode de détection de nanoparticules biologiques à haute sensibilité, comprenant les étapes suivantes :
Étape S1 : réaction d'une nanoparticule de composé de cuivre avec un aptamère de protéine membranaire de surface ayant un groupe sulfhydryle pour obtenir un conjugué composé de cuivre-aptamère de protéine membranaire,
**caractérisé en ce que** :
la nanoparticule de composé de cuivre est une ou plusieurs nanoparticules choisies dans le groupe constitué par le sulfure de cuivre, l'oxyde de cuivre et le sulfure cuivreux;
Étape S2 : filtrer une solution de nanoparticules biologiques contenant les nanoparticules biologiques à travers une première membrane filtrante, et ajouter le conjugué composé de cuivre-aptamère de protéine membranaire à la solution filtrée, pour obtenir un conjugué composé de cuivre-nanoparticules biologiques après réaction ;
Étape S3 : ajout d'un surfactant à la solution de réaction obtenue à l'étape S2, filtrer à travers une seconde membrane filtrante, et lavager la seconde membrane filtrante avec du PBS pour obtenir une membrane filtrante contenant le conjugué nanoparticule biologique-composé de cuivre, la taille des pores de la seconde membrane filtrante étant de 50 nm ; et
Étape S4 : ajout d'une solution d'AgNO₃ à la membrane filtrante obtenue à l'étape S3 et réaction ; puis ajout d'une solution mixte de chlorhydrate de triéthylamine, de peroxyde d'hydrogène et de 3,3',5,5'-tétraméthylbenzidine, réaction pour développement, et observation du changement de couleur de la membrane filtrante à l'oeil nu ou à l'aide d'un appareil photo.

2. Méthode selon la revendication 1, dans laquelle la nanoparticule biologique est un exosome ou un virus.

3. Méthode selon la revendication 1, dans laquelle, à l'étape S1, la taille de la nanoparticule de composé de cuivre est entre 5 et 50 nm, et l'aptamère de protéine membranaire de surface ayant un groupe sulfhydryle est un ou plusieurs aptamères choisis parmi les groupes suivants : l'aptamère CD63, l'aptamère CD81, l'aptamère CD9, l'aptamère EpCAM, l'aptamère HER2, l'aptamère MUC1 et l'aptamère PSMA ; le temps de réaction de la nanoparticule de composé de cuivre avec l'aptamère de protéine membranaire de surface est entre 8 et 24 heures ; et la taille des pores de la première membrane filtrante est 200 nm.

4. Méthode selon la revendication 1, dans laquelle le temps de réaction de la solution de nanoparticules biologiques avec le conjugué composé de cuivre-aptamère de protéine membranaire à l'étape S2 est 0,5 à 10 heures.

5. Méthode selon la revendication 1, dans laquelle le volume de la solution de nanoparticules biologiques à l'étape S2 est réglable, lorsque la concentration de la solution de nanoparticules biologiques est faible, le volume de la solution de nanoparticules biologiques est augmenté pour améliorer la sensibilité.

6. Méthode selon la revendication 1, dans laquelle le surfactant de l'étape S3 est un ou plusieurs agents choisis dans un groupe constitué de : dodécyl sulfate de sodium, bromure de cétyltriméthylammonium, et polyvinylpyrrolidone, et la concentration del surfactant est comprise entre 0,1 et 2,0 %.

7. Méthode selon la revendication 1, dans laquelle la solution d'AgNO₃ à l'étape S4 a une concentration de 10⁻⁵ à 10⁻³ M et un volume de 10 à 50 uL, et le temps de réaction de la substance sur une surface de la membrane filtrante avec la solution d'AgNO₃ est entre 5 et 10 min, et la concentration de chlorhydrate de triéthylamine est 0,05 à 0,2 M, la concentration de peroxyde d'hydrogène est de 0,1 à 0,5 M, la concentration de 3,3',5,5'-tétraméthylbenzidine est de 0,1 à 1,0 mM, et le temps de réaction est de 5 à 30 min.
